# EUROPEAN PATENT APPLICATION

(11) **EP 1 917 968 A1**
(43) Date of publication of application: **07.05.2008**
(21) Application number: 07119792.5
(22) Date of filing: 31.10.2007
(51) Int. Cl.: A61K 35/28, A61P 35/00

(54) **Methods and composition for complementing treatments to eradicate neoplastic cells**

(30) Priority: 31.10.2006 US 855984 P
(71) Applicant: Zech, Herbert, 6900 Bregenz (AT)
(72) Inventor: Zech, Herbert, 6900 Bregenz (AT)
(74) Representative: Dr. Graf & Partner

(57) **Abstract**

Use of a stem cell preparation in the manufacture of a medicament for treatment of a neoplastic disease, wherein the drug is formulated for repopulation of bone marrow after bone marrow ablation in a patient.

## Description

### Background of the Invention

Complete eradication of neoplastic cells and precursors thereof is critical for successful treatment of almost all malignancies, and most common treatment options typically involve a combination of surgical removal/resection, chemotherapy, and/or radiation therapy. Additional therapeutic interventions are typically focused on lifestyle changes, nutrition, etc.

Unfortunately, even where an aggressive treatment protocol is employed, a significant fraction of patients will develop a relapse of the tumor (secondary neoplasm) that is often resistant or less responsive to drug treatment. It is generally believed that secondary neoplasms are either derived from previously undetected neoplastic cell masses, and/or from metastatic cells that may have been present in the circulation and/or in non-malignant host tissue. More recently, it was proposed that secondary neoplasms may possibly also originate from quiescent neoplastic cells or their precursors, which makes eradication with conventional therapeutic approaches difficult as chemotherapy, radiation, and many other current therapies are largely ineffective due to the low metabolism of the quiescent cells. As the quiescent neoplastic cells or their precursors are thought to be single cells, they are systemically difficult to target and/or isolate. To improve efficacy of chemotherapy, several groups reported high-dose chemotherapy for patients with poor prognosis breast cancer and other solid tumors in which autologous or allogenic stem cell infusion was employed to counteract the adverse effects from the chemotherapeutic agents (see e.g., Curr Probl Cancer. 1998 May-Jun;22(3):135-77 or Cochrane Database Syst Rev. 2005 Jul 20;(3):CD003139). However, the treatment results were somewhat inconclusive and did not confirm high expectations.

Stem cells were also implicated in certain neoplastic diseases as a source for neoplastic cells. For example, some groups reported early disseminated breast cancer cells in bone marrow, wherein these cells had putative stem cell phenotype (see e.g., Clin Cancer Res 2006; 12 (19): 5615-5621). Other groups reported transfer of emphysema in a mouse model from a donor strain (Tsk) to control strain (C3H) by transfer of bone marrow from the donor to the control mouse. Remarkably, emphysema was successfully treated in Tsk mice by bone marrow transplant from the control strain to the Tsk strain (see e.g., Stem Cells 2006, 24:2071-2077).

Clearly, numerous methods of treatment to eradicate neoplastic cells are known in the art, however, all or almost all of them suffer from one or more disadvantages. Similarly, stem cells appear to be implicated in various neoplastic diseases, however, therapeutic approaches targeting stem cells and stem cell host tissue have not been developed. Therefore, there is still a need to provide improved compositions and methods to eradicate neoplastic cells and precursors thereof.

### Detailed Description

The inventors have now discovered that recurring neoplastic disease can be prevented in a relatively effective manner by not only eradicating a primary tumor but by also treating the host tissue to eradicate stem cells that may give rise to a secondary tumor. Thereafter, the host tissue is then treated with a stem cell containing preparation to recover from the eradication process.

For example, and in one preferred aspect, the inventors contemplate a method of treating a neoplastic disease in which a patient is treated with surgery, chemotherapy, and/or radiation therapy to eradicate a tumor. In another step, bone marrow is ablated in the patient, and in yet another step, stem cells are administered to the patient, wherein such stem cells were obtained at a point prior to diagnosing the donor/patient with a neoplastic disease.

With respect to the eradication of the primary tumor it is contemplated that all known manners of treatment are deemed suitable, and appropriate treatments will therefore include one or more of surgical removal/resection, chemotherapy, and/or radiation therapy. The person of ordinary skill in the art will readily be able to identify standard treatment options for tumors. It is further preferred (but not necessary) that the eradication of the primary tumor is performed prior to clinical appearance of secondary tumors/metastases, and that the eradication is as complete as possible and/or tolerable. In one especially contemplated aspect, the primary tumor is malignant and is derived (or thought to be derived) from one or more tumor stem cells. Thus, contemplated tumors will include solid tumors and blood-borne neoplasms (e.g., carcinomas, lymphomas, leukemias, sarcomas, mesotheliomas, gliomas, etc.), which may have any stage or grade.

Depending on the particular type of neoplasm, it should be noted that the host tissue may vary considerably, however, it is especially contemplated that the host tissue is the bone marrow. Alternative host tissues may be anatomically defined by structure or function (e.g., lymphatic tissue, dermis, epidermis, muscle) or may be identified by condition (e.g., inflammatory focus, necrotic tissue, or tissue infiltrated by lymphocytes). Therefore, the treatment of the host tissue may vary and a particular treatment will be at least in part be determined by the particular tissue type. However, it is generally contemplated that the treatment is administered under a protocol effective to eradicate tumor stem cells, and especially where such stem cells are quiescent. Such eradication may be performed in single or multiple treatment sessions using the same, different, or combination of treatment modalities (e.g., phototherapy, irradiation, chemotherapy, topical treatment, etc.). For example, and in an especially contemplated aspect, the host tissue is bone marrow and treated by a combination of radiation and chemotherapy to substantially entirely ablate the bone marrow. In another example, where the host tissue is the bladder wall, treatment may include instillation with chemotherapeutic drugs and photosensitizers, which will then be illuminated with light of a wavelength suitable to effect cell death of the targeted tissue. In a further example, where the host tissue is skin, topical treatment may be performed using poration and administration of drugs to the skin (and not, or only in limited quantities to the systemic circulation) at high dosages to eliminate neoplastic stem cells.

Regardless of the manner of host tissue treatment, it should be appreciated that such treatment will not only eradicate neoplastic stem cells and their precursors, but also significantly damage non-neoplastic cells. Therefore, to mitigate such collateral damage, it is contemplated that the treated host tissue (and surrounding tissue, or the entire patient) will receive a stem cell containing preparation in an amount and under a protocol effective to restore the damaged target tissue. There are numerous stem cells known in the art, and all types of stem cells are deemed suitable for use herein. Most preferably, however, it is contemplated that the stem cells that are used for restoration of the host tissue are compatible with the host tissue in their lineage(s) or potential to develop to the appropriate lineage(s). For example, where the host tissue is bone marrow, it is particularly preferred that the stem cell containing preparation includes stem cells that will reconstitute the previously ablated bone marrow. For example, suitable stem cell containing preparations may include previously isolated (and typically cryopreserved) bone marrow, optionally expanded (and typically cryopreserved) cord blood preparations, and other less differentiated stem cell preparations that have the potential to commit to hematopoietic stem cells and/or precursor cells. There are numerous manners of bone marrow reconstitution known in the art, and all of such known manners are deemed suitable for use herein.

Stem cell containing preparations will preferably be enriched in stem cells, and most preferably comprise at least between one and 10³, and more typically between 10³ and 10⁶, and most typically more than 10⁶ stem cells in a formulation suitable for administration. Therefore, suitable stem cells may be optionally pooled native preparations, or at least partially cultured and expanded preparations. Depending on the type of stem cell, administration may be systemic or local (i.e., into the bone marrow matrix), and may be in a single administration or multiple administrations. Furthermore, it is generally preferred that the stem cell of the stem cell preparation is isolated from the same patient from which it was obtained (autologous stem cell), and most preferably at a time before the patient was diagnosed with the neoplastic disease. However, in less preferred aspects, it is also contemplated that the stem cell may be an allogenic or even heterogenic source. In such cases, it is preferred that the stem cell preparation is relatively similar to the host tissue type (e.g., stem cell preparation from bone marrow for treatment of previous bone marrow ablation, stem cells from the cord blood which are more immunotolerant than bone marrow, especially in first grade relatives).

It should further be appreciated that the stem cells for the stem cell preparation can be isolated and/or enriched in numerous manners well known in the art, and suitable manners include FACS, affinity based separation (e.g., using magnetic beads), selective culture, etc. Thus, preparations may be crude (e.g., crude bone marrow or cord blood isolate) or include enriched populations of stem cells (e.g., bone marrow stem cells flushed out and isolated in peripheral blood, or cord blood collected at birth). Depending on the type, isolation, and cultivation conditions, the stem cells in the stem cell preparations will therefore include adult pluripotent stem cells or multipotent hematopoietic stem cells.

Based on these considerations, the inventors contemplate use of a stem cell preparation (e.g., comprising autologous or allogenic stem cells) in the manufacture of a medicament for treatment of a neoplastic disease, wherein the drug is formulated for repopulation of bone marrow after bone marrow ablation in a patient, and wherein the patient has further undergone at least one of surgery, chemotherapy, and radiation therapy as primary treatment of the neoplastic disease. Such stem cell preparations may be prepared in a hospital as adjunct therapy, or more preferably in an outside (commercial) facility that prepares and stores such preparations. Consequently, the inventors also contemplate a method of facilitating treatment for a neoplastic disease in which information is provided to the public that stem cells can be isolated from an individual prior to diagnosis with a neoplastic disease. The stem cells are then processed and stored, preferably in a format suitable for transfusion. In another step, information is provided that treatment of the individual after diagnosis with the neoplastic disease includes bone marrow ablation in addition to at least one of surgery, chemotherapy, and radiation therapy, and in yet another step, the stem cells are released from storage for administration to the individual to repopulate the bone marrow after ablation.

Viewed from a different perspective, the inventors contemplate kits for complementation of treatment of a neoplastic disease that comprise a plurality of human stem cells in a format and quantity suitable for repopulation of bone marrow after bone marrow ablation. Such cells are typically associated with an instruction to administer the stem cells to an individual that has undergone (a) at least one of surgery, chemotherapy, and radiation therapy, and (b) bone marrow ablation. Most typically, association can be performed in written or displayed format, and/or may be in form of an informative brochure of the storage facility.

### Contemplated Examples

The following examples are provided to give a person of ordinary skill in the art guidance and description of one exemplary treatment modality. However, it should be understood many modifications, additions, and deletions could be implemented without departing from the inventive concept described herein.
A patient is diagnosed with breast cancer at an early stage (cT1). After state of the art segmentectomy and sentinel-lymphonodectomy the tumor is staged as being pT1a G1 NO M0. In this situation, most patients recover completely even without any further treatment but there is a live long increased risk for relapse compared to the normal population. The inventors now contemplate that this relapse is mainly due to residing tumor cells (or precursors thereof) in the patient's bone marrow. Thus, ablation of the bone marrow by radiation and/or chemotherapy after surgical treatment will eradicate the residual tumor cells within the bone marrow. After the ablation with e.g. a combination of cyclophosphamid and busulfan followed by total body radiation, stem cells from a cord blood or bone marrow (which comes from a donor or was taken from the patient's hip years before the appearance of the disease and stored in liquid nitrogen) either administered intravenously or directly into the bone marrow will restore the function of the bone marrow completely without the risk of having malignant cells - which are the main source for metastasis and relapse - in this compartment any more. It could well be that patients with advanced stage malignant tumor may profit also from such treatment modalities either by eradicating the residual tumor cells in the bone marrow with prolonged relapse free intervals. In this case repeated bone marrow ablations and stem cell infusions either locally or systematically will help in expanding the recurrence or progression disease free interval. The unit of cord blood or bone marrow administered should -if possible- at least contain 1x10⁶ mononuclear cells/kg and/or 1x10⁵ CD34⁺ cells/kg. Furthermore, the infused fraction can be a mixture of stem cells, such as e.g. 1x10⁶ mononuclear cells/kg and/or 1x10⁵ CD34⁺ cells/kg plus 1x10⁵ mesenchymal stem cells also isolated from the cord blood or bone marrow fraction. If the stem cell source comes from a donor, graft-versus-host disease prophylaxis can for example consist of methylprednisolon and cyclosporine A for a given period of time. CD34 is a cluster of differentiation molecule present on certain cells within the human body. It is a cell surface glycoprotein and functions as a cell-cell adhesion factor. It may also mediate the attachment of stem cells to bone marrow extracellular matrix or directly to stromal cells. Cells expressing CD34 (CD34+ cell) are normally found in the umbilical cord and bone marrow as hematopoeitic cells, endothelial progenitor cells, endothelial cells of blood vessels but not lymphatics (except pleural lymphatics), mast cells, a sub-population dendritic cells (which are factor XIIIa negative) in the interstitium and around the adnexa of dermis of skin, as well as cells in soft tissue tomors.

Thus, specific embodiments and applications of treatments of neoplastic diseases using bone marrow ablation and stem cell reconstitution of same have been disclosed. It should be apparent, however, to those skilled in the art that many more modifications besides those already described are possible without departing from the inventive concepts herein. The inventive subject matter, therefore, is not to be restricted except in the spirit of the present disclosure. Moreover, in interpreting the specification and contemplated claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Furthermore, where a definition or use of a term in a reference, which is incorporated by reference herein is inconsistent or contrary to the definition of that term provided herein, the definition of that term provided herein applies and the definition of that term in the reference does not apply.

## Claims

**1.** Use of a stem cell preparation in the manufacture of a medicament for treatment of a neoplastic disease, wherein the drug is formulated for repopulation of bone marrow after bone marrow ablation in a patient.

**2.** The use of claim 1, wherein the patient has further undergone at least one of surgery, chemotherapy, and radiation therapy as primary treatment of the neoplastic disease.

**2.** The use of claim 1 or 2 wherein the stem cell preparation comprises autologous or allogenic stem cells.

**3.** The use of claim 1, 2 or 3 wherein the stem cells were obtained from cord blood, bone marrow, or peripheral blood that includes bone marrow stem cells flushed from the bone marrow.

**4.** The use of one of claims 1 to 3 wherein the stem cell preparation is enriched in stem cells.

**5.** A method of facilitating treatment for a neoplastic disease, comprising:
providing information that stem cells can be isolated from an individual prior to diagnosis with a neoplastic disease;
processing and storing the stem cells in a format suitable for transfusion;
providing information that treatment of the individual after diagnosis with the neoplastic disease includes bone marrow ablation in addition to at least one of surgery, chemotherapy, and radiation therapy; and
releasing the stem cells for administration to the individual to repopulate the bone marrow after ablation.

**6.** The method of claim 5 wherein the stem cells are cord blood stem cells, bone marrow stem cells, or bone marrow stem cells flushed out and isolated in peripheral blood.

**7.** The method of claim 5 or 6 wherein the step of processing comprises a step of expanding the stem cell population.

**8.** The method of one of claims 5 to 7 wherein the step of releasing the stem cells comprises release of at least 1 stem cell.

**9.** The method of one of claims 5 to 8 wherein the step of processing comprises a step of selectively removing non-stem cells.

**10.** The method of one of claims 5 to 9 wherein the neoplastic disease is a non-hematological cancer.

**11.** The method of one of claims 5 to 10 wherein the step of bone marrow ablation comprises at least one of bone marrow irradiation and chemotherapy.

**12.** The method of one of claims 5 to 11 wherein administration is autologous or allogenic administration of stem cells.

**13.** A kit for complementation of treatment of a neoplastic disease, comprising:
a plurality of human stem cells in a format and quantity suitable for repopulation of bone marrow after bone marrow ablation; and
an instruction to administer the stem cells to an individual that has undergone (a) at least one of surgery, chemotherapy, and radiation therapy, and (b) bone marrow ablation.

**14.** The kit of claim 13 wherein stem cells are cord blood stem cells, bone marrow stem cells, or bone marrow stem cells flushed out and isolated in peripheral blood.

**15.** The kit of claim 13 wherein the stem cells are adult pluripotent stem cells or multipotent hematopoietic stem cells.

**16.** The kit of claim 13 wherein the plurality of stem cells includes at least 1 cell.

**17.** The kit of claim 13 wherein the plurality of stem cells is a previously expanded population.

**18.** The kit of claim 13 wherein the neoplastic disease is a non-hematological cancer.

**19.** A method of treating a neoplastic disease, comprising:
treating a patient with at least one of surgery, chemotherapy, and radiation therapy; additionally ablating bone marrow in the patient; and
administering stem cells from the patient, wherein the stem cells were obtained at a point prior to diagnosing the patient with the neoplastic disease or cord blood provision at birth for this same intention.

**20.** The method of claim 19 wherein the step of ablating is performed using at least one of radiation and chemotherapy.

**21.** The method of claim 19 or 20 wherein the stem cells were previously cryopreserved.

**22.** The method of one of claims 19 to 21 wherein the stem cells were previously expanded.

**23.** The method of one of claims 19 to 22 wherein the stem cells were obtained from cord blood, bone marrow, or peripheral blood that includes bone marrow stem cells flushed from the bone marrow.

**24.** The method of one of claims 19 to 22 wherein the stem cells are adult pluripotent stem cells or multipotent hematopoietic stem cells.
